# EUROPEAN PATENT SPECIFICATION

(11) **EP 1 282 430 B1**
(45) Date of publication and mention of the grant of the patent: **23.11.2005**
(21) Application number: 00990102.6
(22) Date of filing: 20.12.2000
(51) Int. Cl.: A61K 35/74, A61P 37/04

(54) **LIPIDS FOR STIMULATION OF IMMUNE RESPONSE**
LIPIDE ZUR STIMULIERUNG DER IMMUNREAKTION
LIPIDES PUR LA STIMULATION D'UNE REPONSE IMMUNITAIRE

(30) Priority: 29.12.1999 GB 9930862
(43) Date of publication of application: 12.02.2003
(73) Proprietor: PORTER, William Leslie, Diss, Norfolk IP22 1RY (GB)
(72) Inventor: PORTER, William Leslie, Diss, Norfolk IP22 1RY (GB)
(74) Representative: Nash, Keith Wilfrid
(86) International application number: PCT/GB2000/004890
(87) International publication number: WO 2001/049277

(56) References cited:
- WO-A-00/23106
- WO-A-89/04670
- FAUVE R M ET AL: "IMMUNOSTIMULATION WITH BACTERIAL PHOSPHOLIPID EXTRACTS" PROCEEDINGS OF THE NATIONAL ACADEMY OF SCIENCES OF USA, NATIONAL ACADEMY OF SCIENCE. WASHINGTON, US, vol. 71, no. 2, February 1974 (1974-02), pages 573-577, XP000876532 ISSN: 0027-8424
- DATABASE BIOSIS [Online] BIOSCIENCES INFORMATION SERVICE, PHILADELPHIA, PA, US; 1980 JAKONIUK P ET AL: "EFFECT OF PHOSPHO LIPID FRACTIONS FROM SOME BACTERIA AND FUNGI ON MOUSE IMMUNITY TO BACTERIAL INFECTIONS" Database accession no. PREV198172073583 XP002186156 & MEDYCYNA DOSWIADCZALNA I MIKROBIOLOGIA, vol. 32, no. 3, 1980, pages 209-212, ISSN: 0025-8601

## Description

### Field of Invention

This invention relates generally to immune response modulation in man and animals and more especially to lipids to modulate immune response.

### Background to the invention

Control of infectious diseases in man and animals is a subject of major interest and research, since existing means of control have known deficiencies. Thus, antibiotics are continually rendered ineffective by the development of resistant strains of microorganisms, while vaccines are highly specific. Their effectiveness is vulnerable to challenge by organisms of different or altered antigenicity.

One alternative approach is the modulation of the natural or induced immune response, as a means of treating and preventing a wide range of diseases. Since procedures involving parenteral injections entail a risk of injection-site infections, particularly in third world situations, it would be preferable that any such measure should be effective by transmucosal routes.

The present invention concerns the use of lipids, of both bacterial and non-bacterial origin, given to modulate the cellular and antibody response to antigenic challenge, as a means of preventing and treating disease.

It is therefore the principal aim of the invention to present to the body's immune system bacterial lipids, preferably derived from non-pathogenic bacteria, and non-bacterial lipids, whereby to modulate the immune response to antigenic stimulus.

It is known that certain killed bacteria, and extracts thereof, given orally to animals, can enhance immune response to certain test antigens. Antigenicity of certain bacterial lipids, given parenterally, is also known, and an ability to cause immunomodulation has been reported for a few lipid-like chemical structures. However, such substances, as exemplified by lipid A and its derivatives, are lipopolysaccharides and do not conform to the definition of lipids, applicable herein, and as currently given by the International Union of Pure and Applied Chemists, Rules of Organic Molecular Nomenclature (Rules of Lipid Nomenclature).

Enhancement of growth rate in young animals and reduction of the incidence of diarrhoea in animals orally receiving killed bacterial preparations, has also been reported, and it is also known that enhancement of growth rate is achievable in animals receiving extracts of such bacterial preparations adsorbed onto non-lipid particulate matter, as well as in animals receiving similarly administered preparations of whole killed bacteria.

It is also known that antigenic proteins can gain access from the gut to the reticuloendothelial system via lymphoid tissue in the wall of the intestine. It is also known that such materials gain access more readily by this route if they are incorporated in liposomes.

It is further known that lipid molecules comprising a polar head and two or more fatty acid chains may provide antigenic challenge through their presentation to T-cells by association with CD proteins. However, such studies have been confined to the antigenic, rather than immunomodulation, properties of lipids and lipid-like substances. Fauvé and Herin (PNAS USA (1974), 71 (2), 573-577) disclose the effect of injecting bacterial phospholipids into mice, thus increasing their resistance towards infection. Similarly, Database Biosis (Online) Biosciences Information Service, Philadelphia, US (1980) Accession N. PREV198172073583, suggests that intravenously administered bacterial phospholipids significantly increase the resistance of mice to bacterial infection. In addition, Clark *et al* (WO 89/04670) disclose an injectable composition comprising a lipid immunogen and silica. However, no account has been taken of effects of oral administration of lipids, whether or not antigenic in nature, on the cellular or antibody response to administration of different antigens.

### The Invention

The present invention therefore provides for use of lipids, the lipids being defined as esters of glycerol (propane 1, 2, 3-triol) with fatty acids, also including 1, 2-di-O-acylglycerols joined at oxygen 3 by a glycosidic linkage to a carbohydrate, also derivatives of glycerol in which one hydroxy group, commonly but not necessarily primary, is esterified with phosphoric acid as mono- or di-esters and the other two are esterified with fatty acids, thus excluding those organic compounds consisting of a trisaccharide repeating unit with oligosaccharide side chains and acyl or amine linked fatty acids, in the preparation of a composition for use by oral administration, for the stimulation of the immune response.

By one means of manufacture, the bacteria are subjected to a degradation step to render lipid components of the bacterial cells available to receptive areas of the gut wall, as well as the digestive, reactive and absorptive processes of the stomach, by oral administration, the degradation step being controlled so that the particle size of said components, either separately or in physical association with other materials, lies within predetermined limits. The degradation step may typically be by use of an enzyme, by autolysis, by heat treatment such as boiling, autoclaving, irradiation (e.g. gamma radiation) or microwaving, or by chemical treatment.

The present invention provides, in particular, a preparation for oral administration to man and animals, to stimulate the immune response, the preparation containing principally a bacterial (preferably derived from non-pathogenic bacteria) or non-bacterial lipid comprising a polar head and two or more alkyl chains.

The lipids, bacterial or non bacterial, may also be combined with other substances so that the particle size of the combined components lies within predetermined limits.

In accordance with a further feature of the present invention, lipid-containing degraded or partially degraded bacteria or particle-associated extracts thereof, or non-bacterial lipids are given orally in particulate form, the particle size of which is controlled within predetermined limits by:-
a) stopping the enzymatic degradation or autolysis of bacteria at the appropriate point, e.g: by application of heat or other enzyme degrading means; or
b) stopping at the appropriate point heat treatments of bacteria, e.g. boiling or autoclaving or irradiation procedures, or chemical treatment; or
c) attaching or incorporating lipids, preferably comprising beneficially polar heads and two or more alkyl chains, whether or not extracted from bacteria, into or on to particles such as liposomes or other appropriately sized particulate material, such as silica, silicilic acid, kaolin particles, or other such material capable of absorbing or adsorbing lipids or lipid-like substances.

That the particle size is within the predetermined limits may be established by known methods, e.g. microscopic examination or by sizing techniques employing beams or radiation such as laser beams, and by readily available sizing instruments such as the Malvern Mastersizer.

Particle size of lipid-containing degraded or partially degraded bacteria may be at least partially controlled by known centrifugation techniques and further controlled by standard filtration procedures, so that particles of sizes outside the predetermined limits are largely or completely excluded. Particles of differing weights or densities may also be separated by centrifugation, for the same purpose.

The predetermined limits on lipid-containing degraded or partially degraded bacteria particle size may be 0.1 microns to 50 microns and more specifically 0.1 to 25 microns. In particular, 0.2 microns up to 15 microns or even 0.4 up to only 10 microns is preferred, and ideally 0.5 to 8 microns or 0.7 to 5 microns.

The lipid bacterial extracts or lipid-containing degraded bacteria or non-bacterial lipid, whether or not incorporated into liposomes, may be incorporated into particulate formulations of dry or wet powder or granular mixes, where in the latter case the liquid may be water, an edible oil, milk, or any beverage. The degraded bacteria may also be incorporated as suspensions in any of the aforementioned liquids. Powder formulations may also be given in tablets, capsules or paste, or may be incorporated into foods.

The lipid bacterial extracts or lipid-containing partially degraded bacteria may also be mixed with oil, which may be dispersed in water or other suitable liquid with or without the aid of emulsifying agents such as Crillet 4, forming an emulsion in which the bacterial extracts or degraded bacterial product is contained within or bound to the dispersed phase. Oily bacterial extracts may be mixed directly with emulsifying agents to form emulsions.

In the case of carrier particles, these should preferably have a size range of 0.2 to 100 microns, preferably 0.3 to 50 microns and most preferably 0.3 to 15 or even to 10 microns, and ideally 0.5 to 8 microns or 0.7 to 5 microns.

Preferably, in the case of preparations derived from bacteria, after completion of the degradation step, one or more lipid components of the degraded or partially degraded bacteria are extracted. The extracted component or components, or particulate materials to which the component or components are attached, will be of a particle size within the said predetermined limits. Extraction of lipids, e.g. comprising polar heads and two or more alkyl chains can be achieved for example, by treating the bacteria with a chloroform/methanol mixture, although other chemical treatments can achieve the same result.

One feature of the invention thus provides a killed bacterial extract comprising particles of lipids, as defined herein, of dimensions within the above stated predetermined limits.

In another feature of the invention, the immunologically active material employed may be lipids of bacterial or non-bacterial origin, especially compounds comprising a polar head and two or more alkyl chains. They may be phospholipid, glycolipid, or neutral lipids. Such compounds (other than neutral lipids) may consist of a polar head whose molecular weight is within the ranges of 5 to 100 Daltons, or 50 to 150 Daltons, or 100 to 200 Daltons, or 150 to 250 Daltons or 200 to 300 or more Daltons.

The immunologically active material may be formed into liposomes by known techniques, or mixed with oily or viscous preparations to form liposomes, or may be otherwise incorporated into or onto carrier particles of appropriately sized particulate material, such as colloidal silica, silicilic acid, kaolin particles or other such material capable of absorbing or adsorbing lipids. Such particles may be incorporated into powder, tablets, capsules, food, liquids or beverages. The sizes of such liposomes or carrier particles incorporating the immunologically active material may have a size range of 0.2 to 100 microns, preferably 0.3 to 50 microns, most preferably 0.3 to 15 or even 10 microns, and ideally 0.5 to 8 microns or 0.7 to 5 microns.

Three verification examples are now described.

### Example 1

Comparison is made between two tests of immune globulin production in mice, following oral administration of a test antigen, where the mice received daily doses of partially autolysed and autoclaved *Bacillus subtilis*.

In test A, *Bacillus subtilis* was grown in a clear liquid medium containing yeast extract, sucrose, magnesium and other soluble nutrients, according to well-established procedures. The bacterial cells were separated from the culture medium by centrifugation and allowed to stand for 24 hours at room temperature. The cells were examined grossly and microscopically at intervals of about 6 hours. After this period, and before any visible physical breakdown of the bacteria was observed. the cells were autoclaved at 115 degrees C for 20 minutes. Microscopically, the cells all remained substantially intact after autoclaving, with approximate dimensions of 0.5 to 1 x 1 to 5 microns.

The killed bacteria slurry was diluted in water and administered by gastric lavage, daily for 7 days, to 5 mice, at a daily dose rate equivalent to 400 micrograms of bacterial dry matter.

A similar group of 5 mice serving as a control group received daily oral doses of physiological saline.

Both groups were then antigenically challenged with keyhole limpet haemocyanin with cholera toxin. Both groups were then assayed for immune globulins.

The IgA response in the group receiving *Bacillus subtilis* preparation exceeded that in the control group by a factor of 9.

In test B, the above procedure was repeated, except that the *Bacillus subtilis* preparation was allowed to stand at room temperature for a period of 72 hours, so that autolytic changes progressed to a stage of microscopically visible breakdown of the bacterial cells. After the autoclaving, microscopy revealed that the bacterial preparation had broken down to a fine silt, of maximum particle size of 0.2 microns.

The antigenic challenge and subsequent assay of immune globulins was then carried out as in test A.

In test B, IgA response to the bacterial preparation was found to exceed that in the control group by a much reduced factor of 2.

Example 1 thus clearly demonstrates the beneficial effect of a killed bacterial preparation given orally as particles of 0.5 to 5 microns, compared to a similarly administered preparation of sub 0.2 micron particles.

### Example 2

A comparison is made between two separate tests. In test C, chickens of from 1 to 21 days of age received food supplemented with a 2:1 methanol/chloroform extract of *Bacillus subtilis*, at the rate of the extract obtained from 100mg of *Bacillus subtilis* dried biomass per kg of feed.

The methanol/chloroform extract, substantially lipid in nature, was applied to a dusty and finely granular preparation of expanded mica containing a high proportion of particles of approximately 0.2 to 100 microns, before being incorporated into the feed. Growth rate of treated chickens exceeded that of controls by 14.1 per cent.

In test D, mice received a daily dose of a methanol/chloroform lipid extract *of Bacillus subtilis* administered by gastric lavage. The daily dose was the extract obtained from approximately 400mg of dried biomass. Compared to controls, no weight gain enhancement, or enhancement of immune response, was detected.

Example 2 clearly demonstrates the beneficial effect of using an orally administered bacterial preparation or extract in particulate form as compared to a non-particulate form.

### Example 3

*Bacillus subtilis* cells, cultured and subjected to partial autolysis, as in Example 1, test A, were agitated for 48 hours in a mixture of 2 : 1 chloroform and methanol. Following centrifugation, the supernatent was concentrated by evaporation to yield a lipid extract.

The extract was combined with a liposome in a buffered saline solution to give dispersed particles of lipid/liposome of approximately 1 to 3 microns in diameter. This was administered to 5 mice by daily gastric lavage for 5 days, in a total daily dose per mouse of 0.2 ml, giving a total dose per mouse of 0.59 mg of extracted lipid. Five control mice received the same liposome preparation, but without the bacterial lipid extract, suspended in identical buffered saline and also administered at 0.2 ml per mouse per day.

On days 7 and 15, all the mice were immunised by gastric lavage with keyhole limpet haemocyanin (5 mg) in 200 ml of physiological saline containing cholera toxin (10 µg). On day 21, all the mice were bled and mesenteric lymph nodes removed.

Tests by ELISA of specific antibodies in serum showed levels of IgA in the lipid extract treated mice to exceed the control mice by a factor of 8.

Cell cultures of mesenteric lymph nodes showed T cell proliferation in nodes of treated animals in response to keyhole haemocyanin to exceed that in control animals by factors of 35 and 16 following 3 and 5 days of culture.

The results therefore showed marked modulation of immune response through oral pretreatment with bacterial lipid extracts given in panicles of approximately 1 to 3 microns.

It should be noted that the method of extraction employed in Example 3 was specifically selected to extract only lipids from the bacteria. Lipopolysaccharides, such as those described as Lipid A, are not extractable by the method employed.

## Claims

1. Use of lipids, the lipids being defined as esters of glycerol (propane 1,2,3-triol) with fatty acids, also including 1,2-di-*O*-acylglycerols joined at oxygen 3 by a glycosidic linkage to a carbohydrate, also derivatives of glycerol in which one hydroxy group, commonly but not necessarily primary, is esterified with phosphoric acid as mono-or di-esters and the other two are esterified with fatty acids, thus excluding those organic compounds consisting of a trisaccharide repeating unit with oligosaccharide side chains and acyl or amine linked fatty acids, in the preparation of a composition for use by oral administration, for the stimulation of the immune response.

2. A use according to claim 1, comprising use of lipids having a polar head and two or more alkyl chains.

3. A use according to claim 1 or 2, wherein the lipid comprises or is attached to particulate material of predetermined size to facilitate transmucosal administration.

4. A use according to claim 3, wherein the lipid comprises or is attached to particulate material of predetermined size to facilitate oral administration.

5. A use according to any of claims 1 to 4, comprising use of particulate material in the size range 0.1 to 50 microns, preferably 0.3 to 10 microns, most preferably 0.7 to 5 microns.

6. A use according to any of claims 1 to 5 comprising use of phospholipids, glycolipids or neutral lipids.

7. A use according to any of claims 1 to 6, wherein the lipids have molecular weight in the range 5 to 300 Daltons.

8. A use according to any of claims 1 to 8, in which the lipids are attached to silica.

## Patentansprüche

1. Verwendung von Lipiden, wobei die Lipide als Ester von Glycerol (Propan-1,2,3-triol) mit Fettsäuren definiert sind, ebenfalls umfassend 1,2-di-*O*-Acylglycerole, die am Sauerstoff 3 mittels einer glykosidischen Bindung an ein Kohlenhydrat gebunden sind, und ebenfalls Derivate von Glycerol, in denen eine Hydroxygruppe, in der Regel, aber nicht notwendiger Weise, die primäre, mit Phosphorsäure als Mono- oder Diester verestert wird und die beiden anderen mit Fettsäuren verestert sind, wodurch jene organischen Verbindungen ausgeschlossen werden, die aus einer Trisaccharid-Wiederholungseinheit mit Oligosaccharid-Seitenketten und Acyl- oder Amin-verknüpften Fettsäuren besteht, in der Zubereitung einer Zusammensetzung zur Verwendung in oraler Verabreichung zur Stimulierung der Immunreaktion.

2. Verwendung nach Anspruch 1, die Verwendung von Lipiden mit einem polaren Kopf und zwei oder mehr Alkylketten umfassend.

3. Verwendung nach Anspruch 1 oder 2, wobei das Lipid partikuläres Material vorbestimmter Größe umfasst oder an solches gebunden ist, um die transmukosale Verabreichung zu ermöglichen.

4. Verwendung nach Anspruch 3, wobei das Lipid partikuläres Material vorbestimmter Größe umfasst oder an solches gebunden ist, um die orale Verabreichung zu ermöglichen.

5. Verwendung nach einem der Ansprüche 1 bis 4, die Verwendung von partikulärem Material in der Größenordnung 0,1 bis 50 Mikron umfassend, vorzugsweise 0,3 bis 10 Mikron, meistbevorzugt 0,7 bis 5 Mikron.

6. Verwendung nach einem der Ansprüche 1 bis 5, die Verwendung von Phospholipiden, Glykolipiden oder neutralen Lipiden umfassend.

7. Verwendung nach einem der Ansprüche 1 bis 6, wobei die Lipide ein Molekulargewicht in der Größenordnung von 5 bis 300 Daltons haben.

8. Verwendung nach einem der Ansprüche 1 bis 8, in der die Lipide an Siliciumdioxid gebunden sind.

## Revendications

1. Utilisation de lipides, les lipides étant définis comme des esters de glycérol (propane 1, 2, 3-triol) avec des acides gras, incluant également des 1,2-di-*O*-acylglycérols fixés au niveau de l'oxygène 3 par une liaison glycosidique à un hydrate de carbone, des dérivés de glycérol dans lesquels un groupe hydroxy, habituellement mais pas nécessairement primaire, est estérifié avec l'acide phosphorique comme mono- ou di-esters et les deux autres sont estérifiés par des acides gras, excluant ainsi les composés organiques consistant en une unité répétée de trisaccharides avec des chaînes latérales oligosaccharidiques et des acides gras liés à un acyl ou une amine, pour la préparation d'une composition destinée à être utilisée en administration orale pour la stimulation d'une réponse immune.

2. Utilisation selon la revendication 1, comprenant l'utilisation des lipides ayant une tête polaire et deux chaînes alkyles ou plus.

3. Utilisation selon la revendication 1 ou 2, dans laquelle le lipide comprend ou est attaché à un matériau particulaire de taille prédéterminée pour faciliter l'administration transmucosale.

4. Utilisation selon la revendication 3, dans laquelle le lipide comprend ou est attaché à un matériau particulaire de taille prédéterminée pour faciliter l'administration orale.

5. Utilisation selon l'une quelconque des revendications 1 à 4, comprenant l'utilisation d'un matériau particulaire dans la gamme de taille de 0,1 à 50 microns, de préférence 0,3 à 10 microns, de manière encore plus préférée de 0,7 à 5 microns.

6. Utilisation selon l'une quelconque des revendications 1 à 5, comprenant l'utilisation de phospholipides, de glycolipides ou de lipides neutres.

7. Utilisation selon l'une quelconque des revendications 1 à 6, dans laquelle les lipides ont un poids moléculaire dans la gamme de 5 à 300 daltons.

8. Utilisation selon l'une quelconque des revendications 1 à 8, dans laquelle les lipides sont attachés à de la silice.
